Europäisches Patentamt

European Patent Office

Office européen des brevets

(11) Publication number : **0 523 948 A2**

# EUROPEAN PATENT APPLICATION

(21) Application number : 92306415.8

(22) Date of filing : 14.07.92

(51) Int. Cl.⁵ : **C12N 5/08,** A61K 35/14

(30) Priority : **15.07.91 JP 199959/91**

(43) Date of publication of application :
**20.01.93 Bulletin 93/03**

(84) Designated Contracting States :
**DE FR GB**

(71) Applicant : **Nippon Oil Co., Ltd.**
**3-12, Nishishinbashi 1-chome Minato-ku**
**Tokyo 105 (JP)**

(72) Inventor : **Komiya, Koji, c/o Nippon Oil Co. Ltd.,**
**Central Technical Research Lab., 8**
**Chidori-cho,**
**Naka-ku, Yokohama-shi, Kanagawa (JP)**
Inventor : **Takezono, Tetsuya, c/o Nippon Oil**
**Co. Ltd.,**
**Central Technical Research Lab., 8**
**Chidori-cho,**
**Naka-ku, Yokohama-shi, Kanagawa (JP)**

Inventor : **Yabe, Noritsugu, c/o Dept. of**
**Hygiene,**
**Dokkyo Univ. School of Medicine, 880**
**Kitakobayashi**
**Mibumachi, Shimotsuga-gun, Tochigi (JP)**
Inventor : **Matsui, Hisao, c/o Dept. of Hygiene,**
**Dokkyo Univ. School of Medicine, 880**
**Kitakobayashi**
**Mibumachi, Shimotsuga-gun, Tochigi (JP)**
Inventor : **Tsunoda, Takuya,c/o Dept. of**
**Gastroent. Surgery,**
**Wakayama Medical College, 27 Shichibancho,**
**Wakayama-shi, Wakayama (JP)**
Inventor : **Yamaue, Hiroki,c/o Dept. of**
**Gastroent. Surgery,**
**Wakayama Medical College, 27 Shichibancho,**
**Wakayama-shi, Wakayama (JP)**
Inventor : **Tanimura, Hiroshi c/o Dept. of**
**Gastroent. Surgery,**
**Wakayama Medical College, 27 Shichibancho,**
**Wakayama-shi, Wakayama (JP)**

(74) Representative : **Geering, Keith Edwin**
**REDDIE & GROSE 16 Theobalds Road**
**London WC1X 8PL (GB)**

(54) **Method for improving the cancer cell-killing activity of lymphocytes.**

(57)   Human lymphocytes collected from the body of a patient with cancer are cultured in a culture medium containing human lysozyme and cytokine and the resulting lymphocytes are transplanted into the body of the cancerated patient. The human lymphocytes so cultured are useful for vital cell transplant therapy for cancer.

EP 0 523 948 A2

## BACKGROUND OF THE INVENTION

### 1. Field of the Invention

The present invention relates to a method for improving the activity of lymphocytes in killing cancer cells; lymphocytes taken from the human body and so treated can be utilized for killing cancer cells, i.e. for so-called adoptive immunotherapy of cancers, when transplanted into the body of a patient with cancer.

### 2. Description of the Related Art

It is known that cell lines are derived which show the activity of killing cancer cells when peripheral blood lymphocytes obtained from normal people or from a patient with cancer are cultured _in vitro_ in a culture medium to which interleukin 2 (hereinafter referred to as IL-2) has been added. Attempts have been made to conduct a so-called LAK (Lymphokine Activated Killer) therapy, that is to perform therapy against cancer by transplating cells having the activity of killing cancer cells into the body of the patient with cancer.

Such LAK therapy, however, does not offer sufficient effect because LAK cells do not demonstrate sufficiently high specificity to the cancer cells even if the cells having the activity of killing the cancer cells _in vitro_ have been transplanted into the body of the patient with cancer, and because they cause side effects.

As a substitute for LAK therapy, CTL (Cytotoxic T Lymphocytes) therapy is known which involves inactivating suppressor cells present in the living body with the aid of a carcinostatic agent, collecting peripheral blood lymphocytes from a patient with cancer, culturing the peripheral blood lymphocytes together with a self-carcinostatic agent whose functions of fission and multiplication are suppressed by mitomycin C or the like for three or four days, adding IL-2 to the culture medium, culturing the culture medium with the IL-2 added for another three or four days, and administering CTL with activated multiplication into the body of the patient with cancer.

This CTL therapy, however, does not give sufficient carcinostatic effect, although no serious side effects have been recognized.

Hence, recently, so-called TIL (Tumor Infiltrating Lymphocytes) therapy has been attempted. The TIL therapy involves collecting lymphocytes from the site of carcinoma that contains a high density of lymphocytes (killer T cells) attacking the cancer cells specifically, culturing the lymphocytes with IL-2, and transplanting the lymphocytes into the body of a patient with cancer. This TIL therapy, however, is not very effective because the culturing of the lymphocytes requires an extremely long period, e.g. from 30 days to 60 days.

For the above therapies, there may be employed a culture medium for culturing peripheral blood lymphocytes, such as Dulbecco's modified MEM with serum added or serum-free medium (as disclosed in Japanese Patent Publication (kokai) No. 79,969/1990).

## SUMMARY OF THE INVENTION

As a result of extensive research and review on various procedures for further enhancing the anti-cancer effects of conventional LAK therapy, CTL therapy and TIL therapy, it has been found that lymphocytes demonstrating enhanced activity for killing cancer cells can be obtained by culturing lymphocytes collected from the human body in a culture medium containing cytokine and human lysozyme.

The present invention provides a method for improving the cancer cell-killing activity of lymphocytes comprising culturing lymphocytes collected from the human body in a culture medium containing cytokine and human lysozyme.

## BRIEF DESCRIPTION OF THE DRAWINGS

Fig. 1 is a graph showing the effect of human lysozyme upon the cancer cell-killing activity of tumor infiltrating lymphocytes collected from a patient with gastric cancer;

Fig. 2 is a graph showing the effect of human lysozyme upon the cancer cell-killing activity of tumor infiltrating lymphocytes collected from a patient with cancer of the large intestine for; and Fig. 3 is a graph showing the effect of human lysozyme upon the cancer cell-killing activity of tumor infiltrating lymphocytes collected from a patient with cancer of the gallbladder.

## DESCRIPTION OF THE PREFERRED EMBODIMENTS

In accordance with the present invention, lymphocytes derived from the human body are cultured in a culture medium containing human lysozyme and cytokine.

The lymphocytes to be employed for the present invention may include, for example, human cells derived from peripheral blood acute lymphocytic leukemia or human cells derived from lymphocytic tissues such as lymph nodes, pancreas, thymus, bone marrow or lymphoduct. More specifically, the lymphocytes may include, for example, T-cells, e.g. helper cells, suppressor cells or killer cells, B-cells or NK-cells (Natural Killer cells). It is particularly preferred to employ lymphocytes collected from the site of the human tumor because cells (killer T-cells) capable of specifically attacking the cancer cells are present in a high density.

The human lysozyme to be employed in the culture medium for the present invention may be lysozyme collected from tissues of the human body. The human lysozyme is present in the tissues of the human body and secretions therefrom, such as placenta, leukocytes, urine, mother's milk, tears and so on. The human lysozyme is extracted in purity as high as approximately 99% and is commercially available for research purposes.

At the present time, the development of a method for the industrial mass production of human lysozyme has been in progress by taking advantage of the recombinant DNA technique. The Agency of Industrial Science and Technology, MITI, Japanese Government, has already succeeded in expressing the synthetic human lysozyme gene by yeast and in excreting the produced human lysozyme extracellularly, as disclosed in Japanese Patent Publication (kokai) No. 248,488/1987. In addition, the present inventors and the Agency of Industrial Science and Technology have jointly succeeded in improving the production conditions and increasing the quantity of secretion and production of the human lysozyme to a remarkably high extent, as disclosed in Japanese Patent Publication (kokai) No. 234,673/1990. This human lysozyme may also be employed for the present invention.

The cytokine to be employed together with the human lysozyme in the culture medium may include, for example, monokine or lymphokine. As the monokine to be employed for the present invention, there may be mentioned, for example, a macrophage activating factor or a monocyte activating factor. As the lymphokine to be employed for the present invention, there may be mentioned, for example, interleukin 2, interleukin 3, interleukin 4, interleukin 6, a B-cell growth factor, a B-cell differentiation stimulating factor or $\gamma$-interferon. Among the cytokines, interleukin 2 is particularly preferred because it can enhance the cancer cell-killing activity to a higher extent. The cytokines may be added to the culture medium in advance or together with the human lysozyme.

For the method according to the present invention, there is employed a culture medium containing the human lysozyme and the cytokine, in which the human lymphocytes are cultured. The culture medium may additionally contain a growth-stimulating factor such as albumin, insulin or transferrin. The culture medium to be employed may be a serum-containing medium or a serum-free medium, although the serum-free medium is more preferred due to resistance to contamination by mycoplasma or viruses during the culture of the human lymphocytes, readiness of purification after the culture of the human lymphocytes in the culture medium, and improvements in the activity of the human lymphocytes for killing cancer cells.

The serum-free medium preferably employed for the present invention may comprise a serum-free basal medium containing the human lysozyme and the cytokine or containing such a supplemental component as described hereinabove in addition to the human lysozyme and the cytokine. As the serum-free basal medium, there may be employed any conventional culture medium or any modified culture medium as long as animal cells can grow therein. The serum-free basal media may include, for example, ones commercially available under trade marks, such as ASF104 (Ajinomoto Co., Ltd.) and S-Clone SF-02 (Sanko Pure Chemical Industries, Ltd.) as well as NOC-404, NOC-905 and NOC-909 developed by the present inventors. The serum-free basal media such as NOC-404, NOC-905 and NOC-909 are particularly preferable for the method according to the present invention. The major constituents of the basal media NOC-404, NOC-905 and NOC-909 are set forth, respectively, in Tables 1, 2 and 3 below.

As the serum-containing medium, there may be mentioned, for example, Dulbecco's modified MEM medium with human serum added and RPMI-1640 medium with human serum added.

The culture medium to be employed for the present invention may be a liquid culture medium or a solid culture medium.

The concentration of the human lysozyme in the culture medium may be e.g. from 0.1 mg to 2,000 mg, preferably from 0.5 mg to 500 mg, and more preferably from 10 mg to 200 mg per liter of the serum-free medium. The human lysozyme may also be employed in serum-containing culture medium at substantially the same concentration as hereinabove. The concentration of the cytokine may be e.g. from 1 unit to 2,000 units (the unit means JRU (Japanese Reference Unit), hereafter called as unit(s)), preferably from 10 units to 1,000 units, per ml of the culture medium; lymphokine may be employed at substantially the same rate, and interleukin 2 is preferably employed at a rate ranging from 10 units to 1,000 units per ml.

The cytokine may be added to the culture medium together with the human lysozyme or the human lymphocytes may be cultured in a culture medium containing the cytokine, followed by the culture of a culture medium in which the human lysozyme has been cultured.

T A B L E  1: Major Components of Culture Medium NOC-404

| Components | Amounts (mg/liter) |
|---|---|
| Basal media | |
| Eagle MEM | 4,560[1] |
| RPMI-1640 | 5,040[1] |
| Amino acids | |
| L-Arginine HCl | 15 |
| L-Asparagine ($H_2O$) | 15 |
| L-Glutamine | 300 |
| Glycine | 5 |
| L-Proline | 5 |
| L-Serine | 30 |
| L-Threonine | 15 |
| L-Valine | 15 |
| Vitamins | |
| Cyanocobalamin | 0.01 |
| Biotin | 0.01 |
| Pantothenic acid-1/2Ca | 10 |
| Choline chloride | 25 |
| Other organic compounds | |
| d-Glucose | 500 |
| d-Mannose | 100 |
| Sodium pyruvate | 110 |
| Putrescine -2HCl | 0.02 |
| Hypoxanthine | 0.1 |
| Thymidine | 0.025 |
| Ethanolamine | 20 |
| Hormones | |
| Human insulin | 10 |
| 3,3',5-Triiodo-L-thyronine-Na | 0.0065 |
| Metals | |
| Ferric chloride ($6H_2O$) | 5 |
| Copper sulfate ($5H_2O$) | 0.00002 |
| Zinc acetate ($2H_2O$) | 0.00002 |
| Selenous acid | 0.0014 |
| Chelating agents and buffers | |
| Dihydroxyethyl glycine | 815 |
| Glycyl glycine | 1,125 |
| Sodium hydrogen carbonate | 1,400 |

Note: 1) a half of the ordinary concentration

T A B L E  2: Major Components of Culture Medium NOC-905

| Components | Amounts (mg/liter) |
|---|---|
| **Basal media** | |
| Powder medium of mixture of RPMI-1640, Eagle MEM & Dulbecco's modified Eagle | 9,830 |
| **Amino acids** | |
| L-Alanine | 20 |
| L-Glutamine | 300 |
| L-Arginine HCl | 15 |
| L-Asparagine ($H_2O$) | 15 |
| Glycine | 5 |
| L-Proline | 5 |
| L-Serine | 15 |
| L-Threonine | 15 |
| L-Valine | 15 |
| **Vitamins** | |
| Sodium ascorbate | 5 |
| Vitamin $B_{12}$ | 0.000125 |
| Biotin | 0.0025 |
| **Other organic compounds** | |
| Sodium pyruvate | 110 |
| d-Glucose | 100 |
| Choline chloride | 25 |
| Putrescine -2HCl | 0.0125 |
| Hypoxanthine | 0.0025 |
| Thymidine | 0.00125 |
| **Hormones** | |
| Human apotransferrin | 10 |
| Human insulin | 10 |
| **Metals** | |
| Ferrous sulfate ($7H_2O$) | 1 |
| Sodium selenite | 0.0017 |
| **Buffers** | |
| Glycyl glycine | 1,500 |
| Sodium hydrogen carbonate | 1,400 |

5

T A B L E  3: Major Components of Culture Medium NOC-909

| Components | Amounts (mg/liter) |
|---|---|
| **Basal media** | |
| Powder medium of mixture of RPMI-1640, Eagle MEM & Dulbecco's modified Eagle | 9,830 |
| **Amino acids** | |
| L-Alanine | 20 |
| L-Glutamine | 300 |
| L-Arginine HCl | 15 |
| L-Asparagine ($H_2O$) | 15 |
| Glycine | 5 |
| L-Proline | 5 |
| L-Serine | 15 |
| L-Threonine | 15 |
| L-Valine | 15 |
| **Vitamins** | |
| Sodium ascorbate | 5 |
| Vitamin $B_{12}$ | 0.00125 |
| Biotin | 0.0025 |
| **Other organic compounds** | |
| Sodium pyruvate | 110 |
| d-Glucose | 100 |
| Choline chloride | 25 |
| Putrescine 2HCl | 0.0125 |
| Hypoxanthine | 0.025 |
| Thymidine | 0.0125 |
| **Hormones** | |
| Human apotransferrin | 10 |
| Human insulin | 10 |
| Human serum albumin | 2,000 |
| **Metals** | |
| Ferrous sulfate ($7H_2O$) | 1 |
| Sodium selenite | 0.0017 |
| **Buffers** | |
| Glycyl glycine | 1,500 |
| Sodium hydrogen carbonate | 1,400 |

The method according to the present invention can offer advantages for vital cell transplant therapy which has developed and progressed mainly in the U.S. and whose research is also actively in progress in Japan.

The vital cell transplant therapy may include, for example, the adoptive immunotherapy of cancer, autogenous bone marrow graft therapy, autogenous skin fibroblast graft therapy, transplant of fetal Langerhans' islands primary cells to a patient with diabetes mellitus, transplant of fetal neurocyte to a patient with Parkinson's disease, and transplant of fetal liver cells to a patient with hemophilia.

The adoptive immunotherapy of cancer generally involves collecting lymphocytes from the body of a patient with cancer, proliferating the lymphocytes in vitro to enhance the ability to attack cancer cells, and transplanting the lymphocytes into the body of the patient with cancer to kill the cancer cells of the patient. In some cases, cancer cells are also collected from a patient with cancer and cultured together with the lymphocytes collected from the blood of the patient, thereby selectively proliferating killer T-cells which in turn are transplanted into the body of the patient.

These therapeutic techniques can reduce side effects to a lesser level than therapy with a carcino-static agent by selecting appropriate lymphatic cells. Further, these methods are said to be effective for metastatic cancers because the lymphatic cells transplanted are allowed to circulate in the body of the patient with cancer.

The adoptive therapy may be classified into three kinds of therapy as follows.

1. LAK (Lymphokine Activated Killer) cells therapy:

This therapy comprises collecting lymphocytes from the peripheral blood of a patient with cancer, culturing the lymphocytes together with interleukin-2 to activate the LAK cells, and transplanting the activated LAK cells to the body of the patient. This method offers the advantage that the period for the culture of the lymphocytes is as relatively short as three to seven days, however, it suffers from the disadvantages that the resulting LAK cells are less specific to cancer cells and poor in accumulation onto the cancerated tissues, and that the IL-2 should be administered in a large amount together with the activated LAK cells.

2. TIL (Tumor Infiltrating Lymphocytes) therapy:

This therapy involves collecting lymphocytes from a site of carcinoma of a patient with cancer (because killer T-cells attacking specifically the cancer cells are present in a high density at the site of carcinoma), culturing the lymphocytes with IL-2, and transplanting the cultured lymphocytes into the body of the patient. This therapy presents the characteristic that the TIL cells are more active (from 50 to 100 times more active) than the LAK cells employed for LAK cell therapy and have higher specificity and ability to accumulate onto the cancerated tissues than the LAK cells. The TIL therapy, however, presents the problem that the period for culturing the lymphocytes is as extremely long as 30 days to 60 days.

3. CIL (Cytotoxic T Lymphocytes) therapy:

This therapy involves collecting lymphocytes from the peripheral blood of a patient with cancer, mixing the lymphocytes with cancer cells isolated from a site of carcinoma of the patient, culturing the mixture of the lymphocytes with the cancer cells in vitro together with IL-2 to induce killer T-cells, and transplanting the induced killer T-cells into the body of the patient. This therapy can offer the advantages that the resulting killer T-cells are highly specific to cancer cells and high in the ability to accumulate onto the cancerated tissues. On the other hand, the CIL therapy is disadvantageous in terms of the difficulty of isolation of the cancer cells from the site of carcinoma and the necessity of advance administration of an inactivating agent (a carcinostatic agent) into the patient with cancer in order to inactive the suppressor T-cells.

In order to further develop vital cell transplant therapy as described hereinabove, it is required to develop procedures for efficiently growing lymphocytes. More specifically, it is of extreme significance for vital cell transplant therapy to develop technique for growing cells such as lymphocytes, bone marrow cells and skin fibroblast cells in vitro in a culture medium with high efficiency and safety.

It is further to be noted that, although serum-containing culture medium can be employed for the present invention as described hereinabove, the use of serum as a component of the culture medium presents the problems that the culture medium may be contaminated with mycoplasma or viruses, so that it is difficult to provide culture media having a constant quality and to grow cells in a constant fashion, and costs of producing the culture media are rendered expensive. Hence, the use of serum-free culture media is desired in order to avoid the problems that are to be anticipated by the use of serum-containing culture media. It has been noted in producing the serum-free culture media that it is of primary importance to develop growth factors to be employed in the serum-free culture media. The method according to the present invention has succeeded in overcoming the defects and disadvantages inherent in the conventional methods by employing the human lysozyme and the cytokine at the same time as growth factors. The employment of the human lysozyme with the cytokine as the growth factors in the serum-free culture media can yield lymphocytes with high efficiency and enhance the activity of the lymphocytes for killing cancer cells. In these respects, the method according to the present invention can serve as development of vital cell transplant therapy to a great extent.

It is to be noted herein that the method according to the present invention is particularly effective for the TIL (tumor infiltrating lymphocytes) therapy.

The present invention will now be described more in detail by way of examples with reference to the accompanying drawings.

Example 1:

Human lymphocytes were collected from a focus of a patient with gastric cancer and cultured in a RPMI-1640 culture medium containing 10% human serum, to which 200 units/ml of IL-2 (Shionogi, Ltd) had been added. Thereafter, the maintained tumor infiltrating lymphocytes (TIL) were collected from the culture medium and inoculated into RPMI-1640 culture medium containing 10% human serum, followed by culturing the culture medium for four days to yield effect cells.

Then, K562 cells (carcinoma cells obtained from Professor Hiroshi TANIMURA, Department of Gastroenterogical Surgery, Wakayama Medical College, Wakayama Japan) were inoculated into RPMI-1640 culture medium containing 10% human serum, to which 100 $\mu$Ci of $Na_2{}^{51}CrO_4$ (Daiichi Radioisotope K.K.) was added for labelling the K562 cells with $^{51}$Cr. The cells were then cultured in the culture medium at 37°C for one hour, collected from the culture medium, and then washed well. The K562 cells were further floated at a concentration of 1 x $10^5$/ml in the culture medium and employed as target cancer cells.

The effect cells and the target cells were added to a 96-well round-bottom microplate at the rate of 15 x $10^4$/100 $\mu$l of the effect cells with respect to the target cells in the concentration of 1 x $10^4$/100 $\mu$l, i.e. in the ratio of effect cells to target cells (the E/T ratio) of 15 to 1. The microplate was then cultured at 37°C for four hours under a 5% $CO_2$ condition. Thereafter, 100 $\mu$l of supernatant was collected from each well of the microplate and measured for its released activity activity of $^{51}$Cr by the aid of a gamma-counter.

It is to be noted herein that the naturally released activity was computed by measuring the culture medium in the absence of the effect cells and that the maximum released activity was computed by measuring the culture medium to which 100 $\mu$l of 1N HCl was added in place of the effect cells. The percent cytotoxicity (%Cx) was then computed by the following formula:

$$\%Cx = [\text{Experimentally released activity (cpm)} - \text{Naturally released activity (cpm)}]/[\text{Maximum released activity (cpm)} - \text{Naturally released activity (cpm)}] \times 100$$

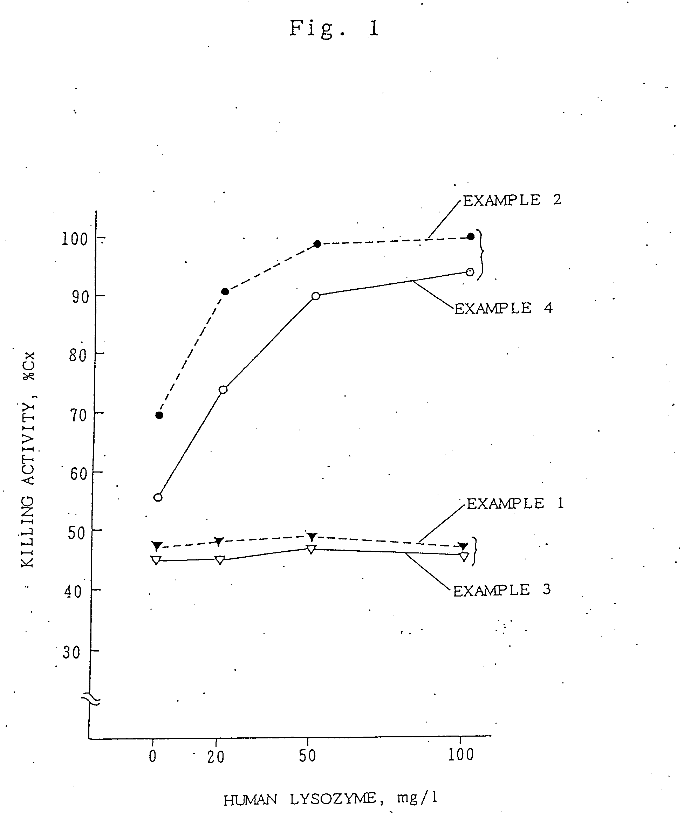

The result is shown in Fig. 1.

Example 2:

The procedures were followed substantially as in Example 1, except for using serum-free culture medium NOC-905 in place of the RPMI-1640 culture medium containing the 10% human serum. The result is shown in Fig. 1.

Example 3:

The procedures were followed substantially as in Example 1, except for using KATO-III cells (carcinoma cells obtained from Professor Hiroshi TANIMURA, Department of Gastroenterogical Surgery, Wakayama Medical College, Wakayama, Japan) as the target cancer cells in place of the K562 cells. The result is shown in Fig. 1.

Example 4:

The procedures were followed substantially as in Example 2, except for using KATO-III cells as the target cancer cells in place of the K562 cells. The result is shown in Fig. 1.

Example 5:

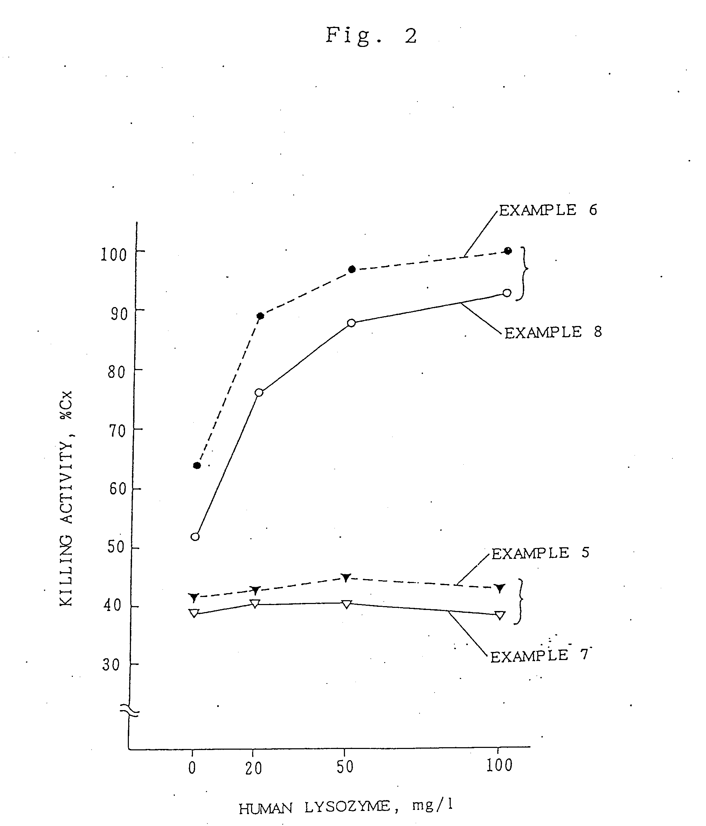

Example 1 was followed in substantially the same manner, except for culturing lymphocytes collected from the focus of the colic cancer of a patient. The result is shown in Fig. 2.

Example 6:

The procedures were followed substantially as in Example 5, except for using serum-free culture medium NOC-905 in place of the RPMI-1640 culture medium containing the 10% human serum. The result is shown in Fig. 2.

Example 7:

The procedures were followed substantially, as in Example 5, except for using KATO-III cells as the target cancer cells in place of the K562 cells. The result is shown in Fig. 2.

Example 8:

The procedures were followed substantially as in Example 6, except for using KATO-III cells as the target cancer cells in place of the K562 cells. The result is shown in Fig. 2.

Example 9:

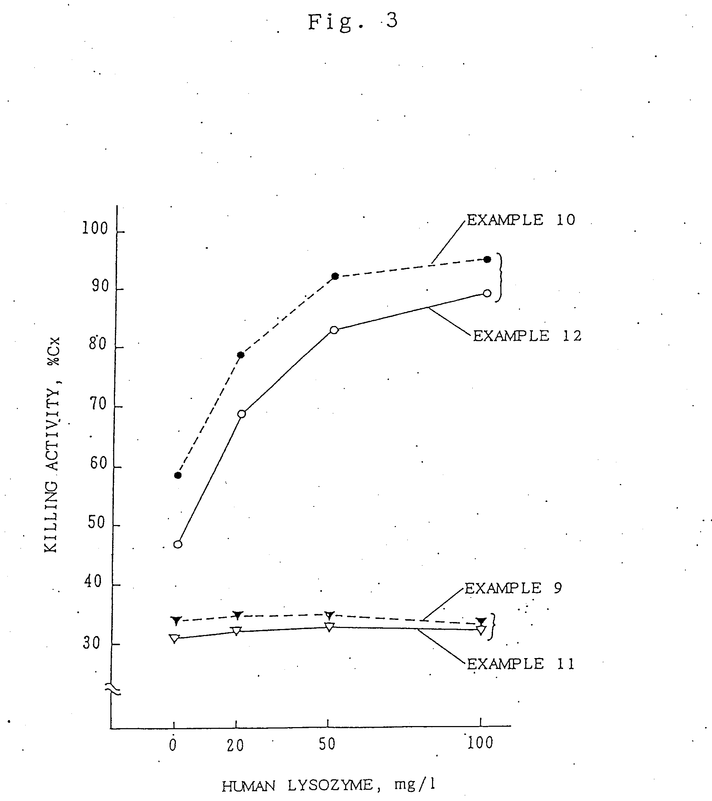

Example 1 was followed in substantially the same manner, except for culturing lymphocytes collected from the focus of the gallbladder cancer of a patient. The result is shown in Fig. 3.

Example 10:

The procedures were followed substantially as in Example 9, except for using serum-free culture medium NOC-905 in place of the RPMI-1640 culture medium containing the 10% human serum. The result is shown in Fig. 3.

Example 11:

The procedures were followed substantially as in Example 9, except for using KATO-III cells as the target cancer cells in place of the K562 cells. The results is shown in Fig. 3.

Example 12:

The procedures were followed substantially as in Example 10, except for using KATO-III cells as the target cancer cells in place of the K562 cells. The result is shown in Fig. 3.

From the results as shown in Figs. 1 to 3, it is apparent that, when lymphocytes were cultured in serum-containing culture media or in serum-free culture media, to which human lysozyme has been added, the activity in killing cancer cells is increased in accordance with the concentration of the human lysozyme and, further, that the activity is enhanced to a remarkably high extent particularly by culture in serum-free culture media.

## Claims

1.  A method for improving the toxic activity of human lymphocytes for killing cancer cells, comprising culturing human lymphocytes collected from the human body in a culture medium containing cytokine and human lysozyme.

2.  A method according to claim 1 wherein said culture medium is a serum-free culture medium.

3.  A method according to claim 1 or 2 wherein said human lymphocytes are human cells derived from peripheral blood acute lymphocytic leukemia or from lymphocytic tissue.

4.  A method according to claim 3 wherein said human lymphocytes are T-cells, B-cells or NK cells.

5.  A method according to any preceding claim wherein said cytokine comprises monokine or lymphokine.

6.  A method according to claim 5 wherein said monokine is selected from macrophage activating and monocyte activating factors.

7.  A method according to claim 5 wherein said lymphokine is selected from interleukin-2, interleukin-3, interleukin-4, interleukin-6, B-cell growth factor, B-cell differentiation stimulating factor and gamma-interferon.

8. A method according to any preceding claim wherein said human lysozyme is extracted from human placenta, leukocytes, urine, mother's milk or tears or is prepared by recombinant DNA technique.

9. A method according to any preceding claim wherein said human lysozyme is contained in the culture medium at a concentration of from 0.1 mg to 2,000 mg per liter of the culture medium, and/or
said cytokine is contained in the culture medium at a concentration of from 1 unit to 2,000 units per ml of the culture medium.

10. A cancer killing human lymphocyte suitable for use in tumor infiltrating lymphocyte therapy, which was cultured in a medium containing lysozyme and cytokine.

## Fig. 1

Fig. 2

Fig. 3